# EUROPÄISCHE PATENTSCHRIFT

(11) **EP 1 341 564 B1**
(45) Veröffentlichungstag und Bekanntmachung des Hinweises auf die Patenterteilung: **15.09.2004**
(21) Anmeldenummer: 01270351.8
(22) Anmeldetag: 11.12.2001
(51) Int. Cl.: A61L 27/34, C09D 5/00

(54) **BIOKOMPATIBLE KOLLOIDE MIT GERINGER ZELLADHÄSION UND AKTIVER STABILISIERUNG VON BLUTPROTEINEN**
BIOCOMPATIBLE COLLOIDS WITH REDUCED CELL ADHESION AND ACTIVE STABILISATION OF BLOOD PROTEINS
COLLOIDES BIOCOMPATIBLES PRESENTANT UNE FAIBLE ADHESION CELLULAIRE ET UNE STABILISATION ACTIVE DE PROTEINES SANGUINES

(30) Priorität: 11.12.2000 DE 10061573
(43) Veröffentlichungstag der Anmeldung: 10.09.2003
(73) Patentinhaber: Max-Planck-Gesellschaft zur Förderung der Wissenschaften e.V., 80539 München (DE)
(72) Erfinder: THÜNEMANN, Andreas, 14469 Potsdam (DE); VIEIRA, Euridice, 80689 München (DE); HERMEL, Horst, 12589 Berlin (DE); MOTSCHMANN, Hubert, Am Mühlberg 1 D-14469 Golm (DE); MÖHWALD, Helmuth, 55411 Bingen (DE); SCHMAHL, Wolfgang, 81247 München (DE); MATIASEK, Kaspar, 80538 München (DE); WERNER, Carsten, 01069 Dresden (DE); SPERLING, Claudia, 01217 Dresden (DE)
(74) Vertreter: Dey, Michael, Dipl.-Chem. Dr.
(86) Internationale Anmeldenummer: PCT/EP2001/014542
(87) Internationale Veröffentlichungsnummer: WO 2002/047738

(56) Entgegenhaltungen:
- EP-A- 0 982 041
- WO-A-99/24513
- DE-A- 19 718 200
- DE-A- 19 913 579
- US-A- 5 443 511
- ANTONIETTI M ET AL: "HYGHLY ORDERED MATERIALS WITH ULTRA-LOW SURFACE ENERGIES: POLYELECTROLYTE-SURFACTANT COMPLEXES WITH FLUORINATED SURFACTANTS" ADVANCED MATERIALS, VCH VERLAGSGESELLSCHAFT, WEINHEIM, DE, Bd. 8, Nr. 1, 1996, Seiten 41-45, XP000551309 ISSN: 0935-9648

## Beschreibung

Die Erfindung betrifft biokompatible Kolloide, die insbesondere in biologischen und medizinischen Anwendungen eingesetzt werden können. Die Kolloide können insbesondere
a) zur Verringerung der Zelladhäsion auf Medizinprodukten und/oder
b) zur Reduzierung pathogener Ablagerungen (Plaques), wie z.B. Amyloidablagerungen,
eingesetzt werden.

Die Verwendung von Werkstoffen in der Medizintechnik wird durch die zum Teil ungenügende Biokompatibilität der verfügbaren Systeme beeinträchtigt. In dieser Hinsicht an Medizinprodukte zu stellende Anforderungen ergeben sich aus Art und Dauer der Anwendung. Anwendungsübergreifend erfordert das Gewährleisten der Biokompatibilität von Materialien und Systemen im Blutkontakt (Blut- oder Hämokompatibilität) aufgrund der raschen systemischen Ausbreitung von koagulatorischen und inflammatorischen Prozessen durch den Blutkreislauf und die implizierte Gefährdung von vitalen Organsystemen besondere Aufmerksamkeit.

Für Medizinprodukte im Blutkontakt (z.B. kardiovaskuläre Implantate, Katheter und Stents, u.a.) sind die wichtigsten materialinduzierten Probleme Thrombogenität, die Aktivierung der Gerinnung sowie humorale und zelluläre Immunreaktionen.² Außerdem sind nicht selten vom Werkstoff ausgehende Infektionen zu konstatieren, die die Morbidität und sogar die Mortalität der betroffenen Patienten erhöhen.³ Die Wechselwirkungen von Blutproteinen, Zellen und Mikroorganismen mit der Materialoberfläche beruhen auf Primärprozessen beim Kontakt des Medizinprodukts mit dem jeweiligen biologischen Milieu. Diese Vorgänge werden hauptsächlich durch die chemische und physikalische Oberflächenstruktur des Werkstoffes bestimmt. Der Schlüssel zu biokompatiblen Materialien liegt also in der Gestaltung der Materialoberfläche.⁴

Eine präzise Zuordnung der für die genannten Blutkompatibilitätsprobleme wichtigen Grenzflächencharakteristika des Materials gelingt jedoch bisher nur in Ansätzen. Hinzu kommt, dass die auf einen einzelnen Aspekt der Hämokompatibilität bezogene Optimierung der Materialeigenschaften oftmals zu Produkten führt, deren Gesamteigenschaften keine praktische Anwendung erlauben. Vielmehr muss eine Balance der günstigen Eigenschaften hinsichtlich verschiedener biologischer Wechselwirkungsprozesse gefunden werden. Bulkmaterialien, die für bestimmte Anwendungen, z.B. für kardiovaskuläre Implantate oder Katheter, in Betracht kommen, müssen ein bestimmtes Materialeigenschaftsspektrum aufzeigen, z.B. Zähigkeit, Elastizität, Festigkeit, Langzeitstabilität, Abriebsstabilität. Diese Eigenschaften sind im Allgemeinen mit entsprechenden polymeren Materialien gut einstellbar und kontrollierbar.

Es wurden auch Techniken entwickelt, um die für medizinische Anwendungen notwendige Reinheit und Sterilisierbarkeit zu erreichen. Diese gewünschten Eigenschaften von Produkten sollen durch Veränderungen zur Verbesserung der Biokompatibilität nicht eingeschränkt werden.

Die Entwicklung völlig neuer Polymerstrukturen, die sowohl die notwendigen Materialeigenschaften als auch eine verbesserte Biokompatibilität aufweisen, wird zwar intensiv betrieben (z.B. segmentierte Polyurethane und verschiedene andere Pfropf- und Blockcopolymere),⁵ ist in der Regel aber sehr kostenintensiv. Oberflächenmodifizierung und Beschichtungsverfahren, die von bekannten Materialien ausgehen, erscheinen in dieser Hinsicht vorteilhaft. Es gibt zahlreiche Ansätze in dieser Richtung. In vielen Fällen versucht man unter Ausnutzung geeigneter funktioneller Gruppen Substanzen oder Polymere auf eine Oberfläche aufzubringen. Entsprechende funktionelle Gruppen können dabei durch nasschemische oder Niederdruck-Plasmabehandlung erzeugt werden oder bereits inhärent im Material vorhanden sein.⁶ Strahlungsinduzierte Pfropfreaktionen, ausgehend von Polymeroberflächen werden erfolgreich praktiziert, jedoch muss dabei beachtet werden, dass die Bildung der funktionellen Gruppen nicht ausschließlich auf der Oberfläche erfolgt und eine Schädigung des Basismaterials stattfinden kann.

Recht einfach realisierbar, jedoch auf bestimmte Materialien beschränkt, ist das Einquellen von z.B. PEO in eine Oberfläche.⁷ Hinsichtlich der durch Oberflächenmodifizierung bzw. Beschichtung einzustellenden Zieleigenschaften zeigen Ergebnisse aktueller Forschung,⁸ dass Materialien aus verschiedenen Palymerfamilien ähnliche Hämokompatibilitätsparameter aufweisen können. Dies lässt auf eine gewisse Adaptionsfähigkeit der involvierten biologischen Mechanismen gegenüber verschiedenen Molekülstrukturen schließen und bildet einen Anknüpfungspunkt für Modifizierungskonzepte. Erfolgreich erscheint in diesem Sinne die Verringerung der Adsorption von Proteinen an PEO-modifizierten Oberflächen und die damit verbundene Vermeidung der Aktivierung von Reaktionskaskaden. Dieser Ansatz wird derzeit intensiv für eine Nutzung in der Medizintechnik geprüft. Allerdings zeigen neuere Untersuchungen,⁹ dass die Konformation und Orientierung der gepfropften, hydrophilen Polymere signifikanten Einfluss auf die gewünschte Minimierung der Adsorption von Proteinen haben.

Bereits seit längerer Zeit werden auch Konzepte verfolgt bei denen durch Einstellung einer negativen Nettoladung oder durch hydrophil/hydrophobe Domänenstrukturen blutkompatible Grenzflächen angestrebt werden.¹⁰ Natürliche Oberflächen, wie etwa die blutkontaktierende Lumenoberfläche von Gefäßen, besitzen zahlreiche molekulare Regulationselemente für die Wechselwirkung mit ihrem Milieu. Erfolgversprechend könnte es deshalb sein, derartige Biomoleküle oder ihre funktionellen Untereinheiten zur Herstellung biokompatibler Materialoberflächen zu immobilisieren oder durch synthetische Strukturen zu imitieren. Dieser Ansatz ist aber entscheidend abhängig vom Erkenntnisstand biochemischer Forschung zu natürlichen Grenzflächenmolekülen. Die Immobilisierung von Proteinen u.a. Biopolymeren¹¹ erwies sich als erfolgreich im Sinne einer zum Teil dramatischen Verbesserung der Hämokompatibilität entsprechend modifizierter Oberflächen. Die Präparation, Handhabung (z.B. Sterilisation) und insbesondere die Dauerhaftigkeit derartiger Modifikationen in der Anwendungssituation muss jedoch kritisch diskutiert werden. Bis auf wenige Ausnahmen (Heparin-Beschichtung von Schlauchsystemen und Oxygenatoren) findet man daher auch durch Biomoleküle veränderte Materialoberflächen noch nicht in der Praxis.

Intensiv verfolgt wird derzeit auch die Einbindung von Phospholipiden in synthetische Polymerstrukturen,¹² Trotz der für diese Materialien beobachteten interessanten Trends darf nicht übersehen werden, dass an Zellmembranen im Blutkontakt Phospholipid-Komponenten kaum in direkten Kontakt mit Blutbestandteilen kommen.

Zur Vermeidung von mikrobiellen Infektionen wurde auch die Ausrüstung von Implantatwerkstoffen mit Antibiotika erprobt.¹³ Materialien, die mit Antibiotika oder Desinfektionsmitteln imprägniert sind, verlieren jedoch offenbar nach kurzer Zeit ihre Wirkung und tragen das Risiko für Resistenzen und Allergien.

Vielversprechende Entwicklungen sind insgesamt auf dem Gebiet des *Tissue Engineering* (Gewebeaufbau durch biochemische Techniken) zu erkennen. Dabei werden abbaubare Polymerstrukturen so gestaltet, dass am Implantationsort das bei der Resorption nachgebildete Gewebe die zu ersetzende Funktion wieder weitestgehend übernimmt.¹⁴ Auch für dieses Konzept ist die Implementierung biologischer Regulationsmoleküle (Z.B. Cytokine, Wachstumsfaktoren etc.) in das synthetische Material wichtig, um die gewünschte Gewebsregeneration zu steuern.¹⁵ Es ist gegenwärtig noch schwer zu erkennen, in welchem Umfang das Prinzip des *Tissue Engineering* Bedeutung für die Bereitstellung von kardiovaskulären Implantaten erlangen wird. Für bestimmte Anwendungsfelder von Medizinprodukten im direkten Blutkontakt (z.B. Membranen für Blutreinigungsverfahren, Katheter, Stents) scheinen Materialentwicklungen auf Basis von *Tissue Engineering* im oben genannten Sinne zunächst nur von untergeordneter Bedeutung zu sein. Problematisch für die Nutzung der genannten Modifizierungs- und Beschichtungsverfahren für etablierte Materialien ist oftmals der erforderliche hohe technologische Aufwand.

Ein wichtiger Aspekt der Biokompatibilität von Materialien ist der häufig auftretende Verlust der biologischen Wirksamkeit von Proteinen durch die Veränderung ihrer Struktur, wenn sie in Kontakt mit den Oberflächen dieser Materialien treten. Die Veränderung der Struktur wird im Allgemeinen als Denaturierung bezeichnet und ist im Wesentlichen auf Konformationsänderungen der Proteine zurückzuführen. Dabei brechen die intramolekulare Wechselwirkungen, die vornehmlich über Wasserstoffbrückenbindungen gebildet werden und damit auch die α-helikale Struktur zusammen, die nahezu alle Proteine zumindest in Teilbereichen aufweisen. Solche Konformationsänderungen werden auch als Falschfaltung bezeichnet.

Weiterhin ist bekannt, dass völlig unabhängig vom Oberflächenkontakt mit exogenen Materialien bestimmte Protein-Spaltprodukte des natürlichen Stoffwechsels der Warmblüter ihre Struktur falschfalten können. Gegenüber ihrer normalen Faltung hat ihre Sekundärstruktur dabei meist deutlich weniger α-Helix- und Random-Coil-Bereiche, dafür aber wesentlich mehr β-Faltblatt-Bereiche. Die Gründe für diese Falschfaltung sind weitgehend unbekannt. Diese an β-Faltblatt-Strukturen angereicherten Strukturen neigen zur Aggregation, bilden unlösliche Fibrillen und akkumulieren zu pathogenen Ablagerungen (Plaques). Die daraus resultierenden Krankheiten werden nach dem stärkemehlartigen Erscheinungsbild der Ablagerungen als *Amyloidosen* bezeichnet, das normal gefaltete Protein als α-Amyloid (αA) und das anomal gefaltete Protein als ,β-Amyloid (βA).

Erfolgen die Ablagerungen im zentralen und peripheren Nervensystem, werden die betroffenen Nervenzellen degeneriert und sterben ab. Findet dieser Vorgang im menschlichen Gehirn statt, ist z.B. die *Alzheimer*-Erkrankung mit zunehmendem Verlust der Hirnleistung die Folge. Zu den Amyloidosen gehören die auch neurodegenerativen Prion-Krankheiten; z.B. *Creutzfeldt-Jakob, Gerstman-Sträussler-Scheinker, Kuru-Kuru* beim Menschen; *Scrapie* beim Schaf und *BSE* beim Rind. Auslöser dieser Erkrankungen sind die auf der Oberfläche von Nervenzellen, vorwiegend denen des Gehirns, sitzenden Prionproteine, die analog den Amyloidproteinen in ihrer normalen Form α-Helix und Random-Coil-reich (Prionprotein cellular, PrPc), in ihrer pathogenen Form β-Faltblatt-reich gefaltet sind (Prionprotein scrapie, PrPsc). Anders als βA kann das anormale PrPsc bei Kontakt mit normalem PrPc dessen Sekundärstruktur zum β-Faltblatt umfalten. PrPsc ist zwischen den Warmblütern übertragbar. Das bedingt unter anderem die Toxizität von BSE und Scrapie belasteten Fleisches in der menschlichen Nahrungskette.

Aminosäuresequenzen von Amyloiden und insbesondere die von β-Amyloid und ihre Synthese in vitro sind bekannt. Ebenso, dass ihre Falschfaltung in die β-Faltblattstruktur und die anschließende Aggregation und Fibrillenbildung in vitro z.B. mit sogenanntem Amyloid(1-43) oder mit Amyloid(1-40) in wässriger Lösung durch Inkubation, z.B. 3 Tage bei 37 °C, erreicht werden kann (Biochemical and Biophysical Research Communications 1994, 205: 1830; Biophysical Journal 1999, 77: 3305).

Außerdem wurden in vitro Versuche durchgeführt, um die Kette αA → βA → Aggregation → Fibrillen zu verhindern oder zu unterbrechen, z.B. durch Reaktion von αA und βA mit Cyclodextrin (Neurobiology of Aging 1992, 13: 595-599), Zerstörung von βA-Aggregaten durch Komplexbildung mit Apolipoproteinen (Biochemistry 1996, 35: 7123-7130; Chemistry and Biology 1996, 3: 949-956; Journal of Biological Chemistry 1997, 272: 18644-18649), Reaktion der βA-Aggregate mit Antibiotika (Rifampicin), Chinonen (Hydrochinon, Benzochinon), natürlichen Radikalfängern (Tocopherol, Ascorbinsäure, Carotin ), speziellen sulfonierten Farbstoffen (Kongorot, Direktrot, Direktgelb) oder Nikotin (Current Medicinal Chemistry 1997, 4: 159-170) oder inhibition der β-Faltblattstrukturierung mit sogenannten "β-sheet breaker peptides" (J. Neurobiol. 1999, 39: 371-382).

Die Versuchsergebnisse zeigten jedoch entweder nicht die gewünschte Wirkung oder die Reagenzien sind für *in vivo* Therapien wegen ihrer Toxizität oder ihrer unerwünschten Nebenwirkungen (z.B. Nikotin) nicht geeignet. Die sogenannten Amyloidosen und insbesondere die neurodegenerativen Amyloid/Prion-Erkrankungen können deshalb nicht therapiert werden und verlaufen, wie allgemein bekannt, allesamt tödlich.

Bekannt ist außerdem der Vroman-Effekt,¹⁶ der besagt, dass die sequenzielle Adsorption einiger Blutproteine an Festkörperoberflächen zumindest teilweise diffusionskontrolliert erfolgt, d.h. die Proteine mit der geringsten Molmasse werden gegenüber größeren zuerst adsorbiert. Die Hauptproteine z.B. im Blut sind Serumalbumin, Immunoglobulin und Fibrinogen mit einer Molmasse von 69000 Da, 160000 Da bzw. 340000 Da. Es kann daher angenommen werden, dass die mit einer Molmasse von vorwiegend < 5000 Da wesentlich kleineren Amyloidmoleküle in der Initialphase des Kontakts mit Blut an Materialoberflächen bevorzugt adsorbiert werden. Analoges gilt generell auch für die anderen Körperflüssigkeiten, Lymphe und Liquor.

Eine Aufgabe der Erfindung war es daher Kolloide mit biokompatiblen Oberflächen bereitzustellen, die in der Lage sind, die native Struktur von Proteinen zu stabilisieren. Die Kolloide sollten dabei medizinisch vertretbare Werte bezüglich ihrer Hämokompatibilität aufweisen, niedrige Werte für Adhäsion von Zellen besitzen und sich in einfacher Weise, z.B. in Form von Folien, Beschichtungen und Nanopartikeln verarbeiten lassen. Darüber hinaus war es eine weitere Aufgabe der Erfindung, therapeutische Mittel bereitzustellen, die konservierend und regenerierend auf die Struktur von Proteinen einwirken. Die Mittel sollten insbesondere auch die Prävention der Amyloid- und Prionprotein-Falschfaltung sowie die Auflösung von Amyloid-Ablagerungen und damit eine Therapie von Amyloid- und Prionerkrankungen bewirken können.

Diese Aufgaben werden gelöst durch eine biokompatible kolloidale Zusammensetzung umfassend
a) mindestens einen Polyelektrolyten, welcher (i) kationische und anionische Gruppen oder/und (ii) Siloxaneinheiten enthält und
b) mindestens ein Fluor-enthaltendes Amphiphil.

Polyelektrolyte sind Polymere mit ionisch dissoziierbaren Gruppen, die z.B. Bestandteile des Grundgerüsts oder/und der Seitenketten oder/und Substituenten der Polymerkette sein können. Erfindungsgemäß bevorzugt ist die Zahl der ionisch dissoziierbaren Gruppen so groß, dass die Polyelektrolyte in der dissoziierten Form wasserlöslich sind.

Amphiphile sind Moleküle, welche sowohl hydrophile als auch lipophile Eigenschaften besitzen, wie etwa Tenside.

Überraschenderweise wurde festgestellt, dass die erfindungsgemäßen Zusammensetzung die Adhäsion von Zellen an Oberflächen reduzieren, die Denaturierung von Proteinen inhibieren bzw. Proteinablagerungen auflösen. PEFA (Polyelektrolyt-Fluoramphiphil-Komplexe), die in den erfindungsgemäßen Zusammensetzungen vorliegen, sowie daraus gebildete Kolloide weisen eine strukturelle Verwandtschaft zu biologischen Systemen, wie etwa Membranen und Vesikeln auf. Durch die Art und Menge an polaren und unpolaren Gruppen kann dabei die Struktur und Funktionalität der Zusammensetzungen, insbesondere von PEFA-Kolloiden bestimmt werden.

Erfindungsgemäß bevorzugt wird ein Amphiphil eingesetzt, welches mindestens eine Trifluormethylgruppe enthält. Es wurde festgestellt, dass bei Verwendung solcher Amphiphile in den erfindungsgemäßen Zusammensetzungen Komplexstrukturen gebildet werden, deren Oberflächen mit Trifluormethylgruppen angereichert sind. Solche Oberflächen weisen eine äußerst geringe Oberflächenergie auf. Die erfindungsgemäßen Zusammensetzungen zeigen bevorzugt eine Oberflächenenergie von < 10 mJm⁻², wobei die Oberflächenenergie z.B. nach Zismann (F. Schulman et al., J. Colloid Sci., 1952, 7, 465) oder nach Neumann (D. Li et al., J. Colloid Interface Sci., 1992, 148, 190) bestimmt werden kann. Während es möglich ist, die Oberflächenenergien der erfindungsgemäßen Zusammensetzungen über einen großen Bereich, etwa von 6 mJm⁻² bis 18 mJm⁻² einzustellen, weisen sie bevorzugt eine Oberflächenenergie von ≤ 14 mJm⁻² und mehr bevorzugt von ≤ 12 mJm⁻² auf. Durch die Verwendung von fluorhaltigen Amphiphilen, deren fluorhaltige Ketten sich an der Oberfläche anreichern, ist es möglich, Materialien mit äußerst geringer Oberflächenenergie, wie etwa ≤ 8 mJm⁻² zu erhalten.

Das erfindungsgemäße Amphiphil weist weiterhin bevorzugt mindestens eine Perfluoralkylgruppierung auf. Besonders bevorzugt wird das Amphiphil ausgewählt aus fluorierten, insbesondere perfluorierten Carboxylaten (z.B. F-(CF₂)ₙ-COO⁻, worin 3<n<17 und bevorzugt 19<n<17), fluorierten, insbesondere perfluorierten Sulfonaten (z.B. F-(CF₂)ₙ(CH₂ₘ-SO₃⁻; worin 3 < n < 13, 0 < m < 8), fluorierten, insbesondere perfluorierten Phosphaten (z.B. 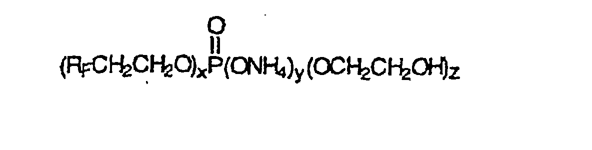 worin x+y+z=3) und Bis(fluorierten Alkyl)-2-sulfosuccinaten (z.B. 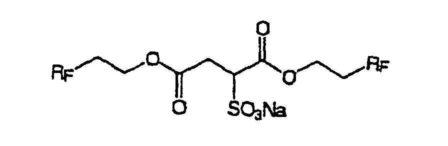 , worin R_{F} jeweils eine fluorierte oder perfluorierte Alkylgruppe, insbesondere mit 1 bis 10 C-Atomen darstellt.)

Überraschenderweise wurde festgestellt, dass die erfindungsgemäßen Zusammensetzungen, obwohl der Polyelektrolyt und das Amphiphil nicht kovalent gebunden sind, gegenüber einem Auswaschen des Amphiphils, beispielsweise durch wässrige Lösungen, Salzlösungen oder Tensidlösungen beständig sind. Eine besonders hohe Stabilität bzw. Beständigkeit wurde bei Einsatz von Amphiphilen beobachtet, die einen fluorierten Rest mit mindestens 10 Kohlenstoffatomen aufweisen. Diese gute Stabilität in Lösungsmitteln ist ein wesentlicher Vorteil gegenüber Oberflächen aus Perfluoralkansäuren auf Platin, welche beispielsweise bei Kontakt mit Wasser leicht aufgelöst werden. Die erhöhte Beständigkeit der erfindungsgemäßen Zusammensetzungen zeigt sich insbesondere im Vergleich zu Mono- bzw. Multischichtoberflächen, die von den gleichen Amphiphilen gebildet werden.

In einer ersten bevorzugten Ausführungsform umfasst die erfindungsgemäße Zusammensetzung mindestens ein Polysiloxan. Auf diese Weise wird ein nicht toxisches Material mit geringer Oberflächenenergie erhalten. Es wurde festgestellt, dass Polysiloxane erfolgreich mit Fluor-enthaltenden Amphiphilen, beispielsweise Perfluoralkansäuren komplexiert werden können, wobei gummiartige Materialien mit äußerst geringen Oberflächenenergien erhalten werden. Das erfindungsgemäß eingesetzte Polysiloxan kann 0,1 bis 100 Mol-% ionische Gruppen enthalten. Die Einheit Mol-% bezeichnet dabei den Anteil an eingesetzten Molen an Monomer, welches mindestens eine ionische bzw. ionisierbare Gruppe in das Polysiloxan einführt, bezogen auf die Gesamtzahl an Molen an eingesetztem Monomer.

Das erfindungsgemäß bevorzugt eingesetzte Polysiloxan kann beispielsweise die Formel 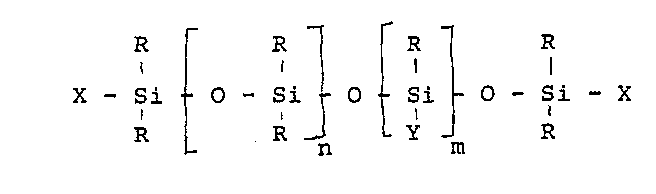 aufweisen, worin R jeweils unabhängig Alkyl, insbesondere C₁-C₄-Alkyl und besonders bevorzugt Methyl darstellt, X jeweils unabhängig Alkyl, insbesondere C₁-C₄-Alkyl und besonders bevorzugt Methyl, oder Hydroxy darstellt, Y einen Rest darstellt, der mindestens eine ionische oder ionsierbare Gruppe enthält, n 0 bis 10.000, bevorzugt 100 bis 5.000 ist, m 1 bis 10.000, bevorzugt 50 bis 5.000 ist und n + m ≥ 50, bevorzugt ≥ 100, mehr bevorzugt ≥ 500.

In einer weiteren bevorzugten Ausführungsform umfasst die erfindungsgemäße Zusammensetzung einen Polyelektrolyten, welcher sowohl kationische als auch anionische Gruppen aufweist. Ein solcher Polyelektrolyt kann auch als Polyampholyt bezeichnet werden. Ein solcher Polyelektrolyt umfasst bevorzugt 5 bis 100 Mol-% ionische Gruppen, mehr bevorzugt von 25 bis 90 Mol-% ionische Gruppen. Das Verhältnis von kationischen zu anionischen Gruppen beträgt bevorzugt 100:1 bis 1:1, mehr bevorzugt 50:1 bis 2:1.

Geeignete kationische Gruppierungen sind beispielsweise quarternäre Amingruppen, geeignete anionische Gruppen sind beispielsweise Carbonsäuregruppen, Phosphorsäuregruppen oder Sulfonsäuregruppen.

In den erfindungsgemäßen Zusammensetzungen liegen der Polyelektrolyt und das Amphiphil bevorzugt in Form von Komplexen vor, wobei insbesondere elektrostatische Wechselwirkungen zwischen den geladenen Gruppen des Polyelektrolyten und geladenen oder polaren Gruppen des Amphiphils ausgebildet sind. Es wurde festgestellt, dass die ausgebildeten Komplexe überraschend erheblich stabiler sind als Monoschichtoberflächen der reinen Amphiphile, sodass eine Auswaschung einer der Komponenten, beispielsweise durch wässrige Lösungen oder Salzlösungen weitgehend vermieden werden kann.

Ein weiterer Gegenstand der Erfindung ist ein Kolloid, gebildet aus der oben beschriebenen Zusammensetzung. Solche Kolloide können z.B. als flüssigkeitskristallartige Strukturen, z.B. in Beschichtungen oder in Filmen, oder als dispergierte Nanoteilchen vorliegen. Es ist bekannt, dass durch Verfilmung aus organischen Lösungsmitteln auf Oberflächen gebildete Kolloide auf der Basis von kationischen Polyelektrolyten und Fluoramphiphilen eine starke Tendenz zur Selbstorganisation besitzen.¹ Auf diese Weise lassen sich auch mit den erfindungsgemäßen PEFA selbst organisierte Strukturen in Form von Oberflächen und Nanopartikeln erzeugen.

Zur Herstellung von Kolloiden in Form von in wässriger Lösung dispergierten Nanoteilchen sind insbesondere Zusammensetzungen geeignet, welche einen Polyelektrolyten mit sowohl kationischen als auch anionischen Gruppen enthalten. Zur Herstellung von Kolloiden in Filmform, beispielsweise als Beschichtung, sind insbesondere Zusammensetzungen geeignet, die als Polyelektrolyten ein Polysiloxan enthalten.

Überraschenderweise wurde festgestellt, dass kolloidale Strukturen, die Oberflächen mit niedriger Oberflächenenergie aufweisen, dadurch hergestellt werden können, dass man einen Polyelektrolyten, welcher kationische und anionische Gruppen oder/und Siloxaneinheiten enthält und mindestens ein Fluor-enthaltenes Amphiphil zusammengibt und das Gemisch auf eine Temperatur von 120 bis 200 °C erwärmt. Die Zugabe eines Katalysators zur Komplexbildung bzw. Vernetzung (Ausbildung von elektrostatischen Wechselwirkungen) ist nicht erforderlich. Das gebildete Kolloid weist eine geringe Oberflächenenergie auf und hat eine hydrophobe und zugleich oleophobe Oberfläche.

Ein Kolloid in Form von in wässriger Lösung dispergierten Nanoteilchen kann dadurch hergestellt werden, dass man einen Polyelektrolyten, welcher insbesondere kationische und anionische Gruppen enthält, mit einem Fluoramphiphilen in wässriger Lösung komplexiert, wobei gleichzeitig Nanopartikel gebildet werden, die in der wässrigen Lösung dispergiert sind. Diese Nanopartikel weisen bevorzugt eine Größe von ≤ 500 nm, mehr bevorzugt ≤ 100 nm und noch mehr bevorzugt eine Größe von ≤ 50 nm auf.

Die erfindungsgemäße Zusammensetzung und das daraus gebildete Kolloid können bevorzugt als Fäulnis-hemmende Mittel, insbesondere zur Reduzierung der Zelladhäsion auf Medizinprodukten, z.B. kardiovaskulären Implantaten und Kathetern, eingesetzt werden.

Ein weiterer Gegenstand der Erfindung ist ein Lackadditiv für einen Polyurethanlack, welches mindestens einen Polyelektrolyten und mindestens ein Fluor-enthaltendes Amphiphil umfasst. Ein solches Lackadditiv kann insbesondere zur Herstellung von Polyelektrolyt-Fluoramphiphil-Kolloid additivierten Polyurethanfolien oder/und Polyurethanbeschichtungen eingesetzt werden.

Überraschenderweise wurde festgestellt, dass PEFA-Kolloide in hohem Maß biokompatibel sind. Die Erfindung betrifft deshalb weiterhin die Verwendung eines solchen biokompatiblen Kolloids, gebildet aus mindestens einem Polyelektrolyten und mindestens einem Fluor-enthaltenden Amphiphil zur Herstellung eines Gegenstandes mit hoher Hämokompatibilität oder/und einer geringen Zelladhäsion. Die biokompatiblen Kolloide, umfassend mindestens einen Polyelektrolyten und mindestens ein Fluor-enthaltendes Amphiphil können weiterhin zur Stabilisierung von Proteinstrukturen, zur Rückfaltung von Proteinen oder/und zur Auflösung von Amyloidablagerungen eingesetzt werden.

Die Erfindung betrifft auch die Bereitstellung und Verwendung von Polyelektrolyt-Fluoramphiphil-Komplexen (PEFAs) in kolloidaler Form. Die erfindungsgemäßen PEFA-Kolloide enthalten sowohl fluorierte, insbesondere perfluorierte aliphatische als auch ionische Gruppen, die in einem räumlichen Abstand von unter 2 nm liegen. Diese PEFA-Kolloide sind hämokompatibel.

Weiterhin sind Gegenstand der Erfindung Geräte, Gegenstände, Dispersionen, nanopartikuläre Formulierungen, die aus den genannten PEFA-Kolloiden bestehen oder Teile aus den genannten PEFA-Kolloiden enthalten und bei deren bestimmungsgemäßer Verwendung diese PEFA-Kolloide mit Blut, Lymphe oder/und Liquor, Proteinen in Berührung kommen, deren Funktion erhalten oder wiederhergestellt werden soll.

Die spezifische Wechselwirkung der erfindungsgemäßen biokompatiblen Kolloide mit falschgefalteten, β-Faltblatt strukturierten Proteinen, vorwiegend Amyloidproteinen und Prionproteinen wird eingesetzt
a. zur Verhinderung und/oder Verzögerung der Umwandlung der Proteinstruktur vom α-Helix-Zustand und Random-Coil-Zustand in den β-Faltblatt-Zustand,
b. zur Proteinstruktur-Rückfaltung vom β-Faltblatt- in den α-Helix- und/oder Random-Coil-Zustand,
c. zur Verhinderung und/oder zur Verzögerung der Aggregation des vorwiegend als β-Faltblatt strukturierten falschgefalteten Proteins,
d. zur Zerstörung und/oder zur Auflösung bereits gebildeter Aggregate in jedweder Form, einschließlich Fibrillen und Plaque,
e. zur gezielten Adsorption von Proteinen an kolloidhaltigen Polymerfolien und/oder an kolloidale Partikel jedweder Größe und Form, z.B. Nanopartikel, zum Zwecke der Peptidentfernung aus lebenden Organismen.

Mit Amyloidproteinen oder/und Prionproteinen assoziierte Erkrankungen sind z.B. Alzheimer, Creutzfeldt-Jakob, Gerstmann-Sträussler-Scheinker, Kuru-kuru, Scrapie und BSE.

Erfindungsgemäß wird die Therapie der dargestellten Erkrankungen ermöglicht, indem
a. das αA und/oder βA sowie PrPc und/oder PrPsc durch Adsorption an die erfindungsgemäßen Kolloide gebunden und in diesem Zustand aus dem natürlichen Stoffwechselkreislauf entfernt werden, z.B. durch natürliche Ausscheidung, Bindung an Implantate oder Blutwäsche,
b. βA und/oder PrPsc an die erfindungsgemäßen Kolloide adsorbiert werden, in diesem Zustand die β-Faltblattstruktur rückfaltet und/oder die Sekundärstruktur gänzlich zerstört wird und anschließend gegebenenfalls das Protein wieder desorbiert,
c. das PEFA-Kolloid an bereits ausgebildete βA- und/oder PrPsc-Aggregate, Fibrillen und/oder Plaques gebunden wird und diese zerstört.
wobei a), b) und c) im lebenden Organismus ohne Schädigung desselben ablaufen sollen.

Der Einsatz der erfindungsgemäßen Kolloide für Therapiezwecke kann bevorzugt erfolgen als Folie (z.B. für Implantate oder Blutwäsche), als Lösung oder feinteilige Dispersion (für eine direkte Injektion) oder feinteilig gekapselt (z.B. für eine Injektion zur Überwindung der Blut/Hirn-Schranke).

Die PEFA-Kolloide weisen insbesondere folgende vorteilhafte Eigenschaften auf:
1. Die Biokompatibilitätswerte von PEFAs bezüglich ihrer Thrombogenität; Immunogenität und Sensibilisierung im Kontakt mit Blut liegen im oder über dem Bereich von Polymermaterialien, die in medizinischen Anwendungen im Dauerkontakt mit Blut umfangreiche Verwendung finden. Dies lässt auf eine hohe Blutverträglichkeit der PEFA-Kolloide schließen.
2. Die Zelladhäsion, z.B. von Blutzellen, an PEFA-Kolloiden ist gering.
3. PEFA-Kolloide stabilisieren die Sekundärstruktur von Blutproteinen.
4. PEFA-Kolloide sind in der Lage die β-Faltbiattkonformation des Amyloid- und Prionproteins, welche der Auslöser für die Evolution der Amyloidosen und Prionenerkrankungen ist, in die α-helikale und random coil Konformation zurückzufalten, die nicht krankheitsauslösend sind.
5. PEFA-Kolloide sind in der Lage Amyloid-Ablagerungen aufzulösen.

Durch die Kombination dieser vorteilhaften Eigenschaften mit der weitreichenden chemischen und strukturellen Variationsmöglichkeit der PEFA-Kolloide, der einfachen Einarbeitbarkeit von PEFA-Kolloiden in Beschichtungen, Polymerfilme und Lacke sowie der Herstellung von PEFA-Nanopartikel eröffnet sich für diese Kolloide eine breite Palette von biologischen und medizinischen Anwendungen.

### BEISPIELE

### 1. Herstellung von PEFA-Kolloiden

### 1.1 Niederenergetische Polysiloxankomplexe

### 1.1.1 Filmherstellung

20,83 g aminofunktionalisiertes Polysiloxan (WR1100, Wacker Chemie, Burghausen) wurden in 100 ml Hexan gelöst. Eine äquimolare Menge an Perfluordodekansäure wurde in einer Mischung aus 50 ml 2-Butanol und 0,5 ml Methanol gelöst. Die Stöchiometrie bezieht sich dabei auf das Verhältnis von Carbonsäuregruppen der Säure zu den Amingruppen des Polysiloxans. Die Lösung der Perfluordodekansäure wurde so langsam unter Rühren zur Lösung der Polysiloxans zugegeben, dass eine transparente und homogene Komplexlösung entstand. Diese Komplexlösung wurde auf Glas, Aluminiumblech und Polytetrafluorethylen als Trägermaterial aufgetragen. Nach Verdampfen des Lösungsmittels wurde der Komplex bei 180°C ohne Zusatz von Katalysator in Gegenwart von Luftsauerstoff vernetzt. In gleicher Weise wurden 0,5 Äquivalente Perfluordodekansäure (Komplex PF12-1.0:0.5), 1,0 und 0,5 Äquivalente Perfluoroctansäure (Komplexe PF8-1.0:1.0 und PF8-1.0:0.5) sowie 1,0 und 0,5 Äquivalente des kommerziell eingesetzten Fluortensids Zonyl FSA (DuPont, FSA-1.0:1.0 und FSA-1.0:0.5) eingesetzt und zu Komplexoberflächen verarbeitet.

### 1.1.2 Oberflächenenergien

Zur Bestimmung der Oberflächenenergien wurden die dynamischen Kontaktwinkel von Testflüssigkeiten unterschiedlicher Oberflächenspannung mit Hilfe der Sessile-Drop-Methode bestimmt.¹⁷ Vor den Messungen wurden die Oberflächen für 24 h in fließendem destilliertem Wasser gelagert um sicherzustellen, dass eventuell vorhandene Spuren von nicht komplexierter Dodekansäure entfernt werden. Daten für die *advancing* Kontaktwinkel von Komplexen mit einem Komplexierungsgrad von 100, 50 und 0 %, entsprechend einem Verhältnis von Aminogruppen zu Carbonsäuregruppen von 1:1, 1:0,5 und 1:0, sind in Tabelle 1 dargestellt. Die Oberflächen des nicht komplexierten Polysiloxans sind hydrophob (θ = 98°), jedoch nicht oleophob, da Dekan und Dodekan auf den Oberflächen spreiten (θ = 0°). Die Hydrophobizität nimmt mit steigendem Fluoranteil (steigender Komplexierung) zu und erreicht ein Maximum der ÖI- und Wasserabweisung bei einem Grad der Komplexierung von etwa 50 %. Die Kontaktwinkel von Wasser als Testflüssigkeit betragen 130° für einen Komplexierungsgrad von 50 % und 131 ° für einen Komplexierungsgrad von 100 %. Die entsprechenden Werte für Hexadekan als Testflüssigkeit betragen 84° und 85°, die für Dekan 75° und 78°.

Zum Vergleich: Zisman et. al. bestimmten für Perfluordodekansäure, die an Platinoberflächen adsorbiert war, einen Kontaktwinkel von 105 ° für Wasser. Dabei wurde gefunden, dass die Oberfläche von Wasser aufgelöst wurde. Die Kontaktwinkel für Hexadekan und Dekan waren dort 78° und 70°. Die erfindungsgemäß beschichteten Oberflächen weisen somit gegenüber den Monoschichtoberflächen des Standes der Technik deutlich erhöhte Kontaktwinkel auf. Darüber hinaus waren die Schichten von absorbierter Perfluordodekansäure gemäß der Erfindung gegenüber den untersuchten Testflüssigkeiten stabil. Mit Hilfe der Elektronenmikroskopie wurde festgestellt, dass die Oberflächen der Komplexe eben im Längenskalenbereich bis deutlich unter 1 µm sind. Somit können Oberflächenrauigkeiten als Ursache für die hohen Kontaktwinkel ausgeschlossen werden.

Auf der Basis der Kontaktwinkelmessungen wurden auch die Oberflächenenergien nach unterschiedlichen Methoden bestimmt. Zum einen erfolgte die Bestimmung der Oberflächenenergien nach der Methode von Neumann und Li.¹⁸ Wie in Tabelle 1 zu sehen ist, liegen die danach bestimmten Oberflächenenergien zwischen 6,8 und 9,9 mN/m für Komplexierungsgrade von 50 und 100 %, was erheblich unterhalb der Werte liegt, die für das nicht komplexierte Polysiloxan gefunden wurden (24,3 bis 26,3 mN/m).

Zum zweiten wurden die kritischen Oberflächenspannungen y_{c} nach Zisman bestimmt.¹⁹ Die danach bestimmten kritischen Oberflächenspannungen betragen ca. 6 mN/m für Komplexierungsgrade von 50 und 100 %. Es sei an dieser Stelle darauf hingewiesen, dass die kritische Oberflächenspannung einen rein empirischen Parameter darstellt, der keine physikalische Bedeutung besitzt, der allerdings seit vielen Jahren routinemäßig für die Charakterisierung von fluorierten Oberflächen eingesetzt wird.

Als dritte Methode zur Bestimmung der Oberflächenspannung wurde die Methode nach Girifalco-Good-Fowkes-Young^{20,21} verwendet. Diese ergibt den dispersen Anteil an der Oberflächenenergie yₛ^{d}. Die für yₛ^{d} bestimmten Werte betragen 9,0 mN/m und 8,4 mN/m für Komplexierungsgrade von 50 % und 100 %.

Die Kontaktwinkeldaten und Oberflächenenergien lassen sich dahingehend interpretieren, dass die Komplexoberflächen mit CF₃-Gruppen gesättigt sind, wenn der Grad der Komplexierung 50 % übersteigt (vgl. Beispiel 1.1.3, Oberflächennahe Elementzusammensetzung).

Zum Test der Komplexstabilität wurden die Oberflächen für 24 h in Tauchbädern mit verschiedenen Flüssigkeiten umspült: a) 1 gewichtsprozentige wässrige Natriumdodecylsulfatlösung, b) Methanol und c) 3 gewichtsprozentige Natriumchloridlösung. Wie in Tabelle 1 zu erkennen ist, reduzieren sich die Kontaktwinkel der Oberflächen der Komplexe nach Behandlung mit Natriumchloridlösung nur unwesentlich. Für die Komplexoberflächen, die Methanol ausgesetzt waren, wird eine signifikante Reduzierung der Kontaktwinkel gefunden. Diese liegen allerdings noch deutlich oberhalb derjenigen, die für die Oberflächen des nicht komplexierten Polysiloxans erhalten werden.

Die Oberflächenenergien, bestimmt nach der Methode von Neumann und Li¹⁸ liegen zwischen 8,1 und 13,5 mN/m, die yₛ^{d}-Werte betragen 9,9 bis 11,5 mN/m. Aufgrund der niedrigen Oberflächenenergien wird gefolgert, dass die Komplexoberflächen auch nach Lösungsbehandlung noch stark an CF₃-Gruppen angereichert sind. Weder Tensid noch Salzlösung löst die Perfluordodekansäure aus der Komplexoberfläche heraus. Demgegenüber kommt es durch die Behandlung mit Methanol zu einem partiellen Auswaschen von Perfluordodekansäure aus der Komplexoberfläche.

**Tabelle 1.**

| Kontaktwinkel von Testflüssigkeiten auf Filmen von Polysiloxankomplexen mit Perfluordodekansäure, vernetzt für 12 h bei 125 °C. Die Filmdicke beträgt 1 mm. Das Verhältnis von Aminofunktionen zu Carbonsäuregruppen ist 1:0, 1:0,5 und 1:1. Die Oberflächenenergien *y*_{*s*} wurden nach der Methode von Neumann und Li bestimmt.¹⁸ | | | | | | | |
|---|---|---|---|---|---|---|---|
| Komplexierungsgrad Fluoranteil in Gew.-% | | 0 | 50 % | | | | 100 % |
| | | 0% | 4,58% | - | | | 8,76 % |
| nach Lagerung in | | Wasser | Wasser | SDS | Methanol | NaCl | Wasser |
| Testflüssigkeit | *y*_{*1*} [mN/m] | *θ(y*_{*s*}*)* [°] [mN/m] | *θ(y*_{*s*}*)* [°] [mN/m] | *θ (y*_{*s*}*)* [°] [mN/m] | *θ (y*_{*s*}*)* [°] [mN/m] | *θ(y*_{*s*}*)* [°] [mN/m] | *θ(y*_{*s*}*).* [°] [mN/m] |
| Decan | 23,9 | 0 | 75 (9,9) | 70 (11,2) | 61 (13,5) | 69 (11,2) | 78 (9,2) |
| Dodecan | 25,4 | 0 | 79(9,6) | 75 (10,6) | 69(12,2) | 73 (11,2) | 82 (8,8) |
| Hexadecan | 27,6 | 19 (26,3) | 84 (9,2) | 83 (9,4) | 80 (10,2) | 76 (11,4) | 85 (8,9) |
| CH₂I₂ | 50,0 | 71(25,5) | 115 (6,7) | 107(9,4) | 103(11,0) | 107(9,4) | 114(7,0) |
| Wasser | 72,7 | 98(24,3) | 130(6,8) | 127 (8,1) | 122(10,5) | 125(9,1) | 131 (6,4) |
| Disperser Anteil der Oberflächenenergie yₛ^{d} [mN/m] | | - | 9,0±0,3 | 9,9 ±0,6 | 11,5 ±0,9 | 10,8-±0,1 | 8,4±0,1 |
| Der Messfehler für die *advancing* Kontaktwinkel θ beträgt ca. 1°, die Oberflächenenergien *y*_{*s*} wurden auf der Basis der *advancing* Kontaktwinkel nach der Methode von Neumann and Li¹⁸ berechnet. | | | | | | | |

Die Empfindlichkeit der Kontaktwinkel auf die chemische Zusammensetzung der Oberfläche liegt bei wenigen Atomlagen. Dabei haben polare Flüssigkeiten wie Wasser eine größere Empfindlichkeit auf tieferliegende Atomlagen als unpolare Flüssigkeiten.^{22,23} Um die oberflächennahe Elementzusammensetzung zu bestimmen, wurden Röntgenstrahl-Photo-Elektronenspektroskopische Messungen (XPS) durchgeführt. Die Analysentiefe dieser Methode liegt unterhalb 5 nm für einen take-off-Winkel von 20°. Es wurde gefunden, dass die Elementzusammensetzung der gemäß Beispiel 1.1.1 hergestellten Beschichtungen in der oberflächennahen Region beträchtlich von der mittleren Komplexzusammensetzung abhängt. So wurde ein beträchtlicher Überschuss von Fluor an der Oberfläche gefunden. Der Fluoranteil an der Oberfläche betrug 32,5 Atom-%, ein Wert, der um den Faktor 5,2 oberhalb der durchschnittlichen Werte liegt (6,2 Atom-%). Da die Analysentiefe des XPS größer ist als die Länge der Fluorkette von Polyfluordodecansäure (ca. 1.5 nm) würden die Elemente Stickstoff und Silicium die von der Polyelektrolytkomponenten stammen auch im Fall einer dicht gepackten Monolayer von Perfluordodekansäure an der Oberfläche gefunden werden. Die gemessene oberflächennahe Konzentration von Stickstoff und Silicium betrug 2,0 und 11,4 Atom-%, während die theoretischen Werte für eine homogene Zusammensetzung bei 0,5 und 22,1 Atom-% liegen. Der in der oberflächennahen Schicht gefundene höhere Stickstoffanteil ist konsistent mit einer Komplexstruktur die dadurch entsteht, dass die fluorierten Ketten an die Komplexoberfläche migrieren. Dabei folgt jeder fluorierten Kette ein Ammonium-Carboxylat-lonenpaar. Offensichtlich befindet sich der weitaus überwiegende Teil der lonenpaare nicht direkt an der Komplexoberfläche sondern in einem Abstand von größer gleich 1 nm. Dies erklärt die hohen Kontaktwinkel gegenüber Wasser. Erwartungsgemäß ist eine Anreicherung von fluorierten Gruppen an der Komplexoberfläche von einer Reduzierung des Anteils an Siloxangruppen begleitet.

### 1.1.4 Katalysatorfreie Vernetzung

Die Vernetzung der Komplexe zu Filmen wurde ohne Katalysator durchgeführt. Qualitativ wurde gefunden, dass die Vernetzung der Komplexe ohne Katalysator bereits bei vergleichsweise niedrigen Temperaturen von 125 °C durchgeführt werden kann. Weiterhin wurde gefunden, dass diejenigen Komplexe, die freie Aminogruppen tragen, schneller vernetzen als diejenigen, bei denen ein Überschuss an Säure zugegeben wurde. Dies belegt, dass die Beschleunigung der Vernetzung nicht allein auf die Säuregruppen zurückgeführt werden kann. Eine maximale Vernetzungsgeschwindigkeit wurde für einen Komplexierungsgrad von ca. 50 % gefunden. Mit Hilfe zeitabhängiger dynamisch-mechanischer Messungen konnte dies bestätigt werden. Der Anstieg des Speichermoduls im Verlauf der Vernetzung des Komplexes ist vergleichbar mit derjenigen Vernetzung des nicht komplexierten Polysiloxans mit einem Schwermetallkatalysator.

### 1.2 PEFA-Kolloid additivierte Polyurethanfolien und -beschichtungen

Die Herstellung von PEFA-Kolloid additivierte Polyurethanfolien und - beschichtungen verläuft in drei Schritten: Erstens die Herstellung einer geeigneten PEFA-Substanz, zweitens die Herstellung eines PEFA-haltigen Lackadditivs und drittens der Verarbeitung des PEFA im Polyurethan-Lacksystem zu Folien und Beschichtungen.

### 1.2.1 Komplexherstellung (PEI-800-Zonyl FSA):

15 mmol Polyethylenimin (0.645 g, M_{w} = 800 g/mol, BASF) werden in 63,4 ml Wasser (Millipore) gelöst und mit 10 % HCl auf pH 3 angesäuert. In diese Polyelektrolytlösung werden unter starkem Rühren innerhalb von 10 min 7,5 mmol (13,6 g, 25 Gew.-% Lsg) Zonyl FSA getropft, das in 102 ml Wasser gelöst ist. Der pH-Wert soll anschließend < 6.8 liegen. Der gebildete PEI-Zonyl FSA-Komplex fällt als Feststoff aus, der abgetrennt und viermal mit 160 ml Wasser (Millipore) gewaschen wird. Die elektrische Leitfähigkeit des Waschwassers sollte nach dem letzten Waschgang maximal 10 *µ*S betragen. Die Ausbeute an Komplexmaterial nach 12 h Trocknung im Vakuum bei 50 °C beträgt 3,45 g (85 % der Theorie).

### 1.2.2 Herstellung eines PEFA-haltigen Lackadditivs:

5 g fein gepulverter PEI-Zonyl FSA-Komplex werden in 5 g Dimethylaminoethanol (DMAE) gelöst. Anschließend wird die Komplexlösung mit 20 g 2-Propanol homogen verrührt und abschließend mit 20 g PUR-Verdünnung (Relius, im Wesentlichen Xylol) verdünnt. Auf diese Weise wird ein gebrauchsfertiges PEI-Zonyl FSA Lackadditiv mit 10 %-igem Feststoffanteil erhalten.

### 1.2.3 Verwendung von PEFA in 2K PUR-Lack

In 64,14 g der OH-Komponente des 2K PUR-Systems Oldodur ADN (Relius) werden 0,86 g des Lackadditivs eingemischt. Diese werden anschließend mit 25,68 g der NCO-Komponente vermischt und in üblicher Weise durch Rollen oder Sprühauftragung verarbeitet.

### 1.3 Herstellung von PEFA-Nanopartikeln

Die Herstellung von Komplex-Nanopartikeln erfolgt in drei Stufen: 1. Synthese von Precursorpolymeren, 2. Quaternisierung der Precursorpolymere zu Polyelektrolyten sowie 3. die anschließende Komplexierung der Precursorpolyelektrolyte bei gleichzeitiger Dispergierung zu PEFA-Nanopartikeln.

### 1.3.1 Synthese der Precursorpolymeren

Precursorpolymere wurden aus Vinylbenzylchlorid und Methylmethacrylat mittels der freien radikalischen Polymerisation in Dioxan synthetisiert, so dass die Polymerisationsgrade bei etwa 500 lagen. Dazu betrug die Gesamtmonomerkonzentration jeweils 4,0 Mol I⁻¹. Als Starter wurde Dibenzoylperoxid mit einer Starterkonzentration von 0,02 Mol I⁻¹ verwendet. Die Reaktionszeiten betrugen 90 min bei einer Temperatur von 70 °C. Polymerisiert wurde in einer Schutzatmosphäre aus Argon. Die unter diesen Reaktionsbedingungen entstandenen Precursorpolymere wurden aus dem Reaktionsansatz mit Methanol gefällt und durch einmaliges Fällen aus Butanon gereinigt. Bei Monomeranteilen an Vinylbenzylchlorid von 12, 25, 50 und 100 Mol-% betrug der entsprechende Vinylbenzylchloridanteil im Polymer 29, 57, 79 und 100 Mol-% (bestimmt mittels ¹H-NMR-Spektroskopie). Mit Hilfe der Gelpermeationschromatographie (THF, Polystyrol-Standards) wurden die Molmassen zu Mₙ = 43000, 42500, 40000, 50000 g Mol⁻¹ bestimmt bei Polydispersitäten von M_{w}/Mₙ = 1,72, 1,62, 1,69 und 1,69. Die mit Hilfe der Differenzialkalorimetrie bestimmten Glaspunkte der Precursorpolymeren betrugen 110, 101, 92 und 77 °C.

### 1.3.2 Quaternisierung der Precursorpolymere zu Polyelektrolyten

Die Chlormethylfunktion der Precursorpolymere wurde in einer Heterophasenpolymerisation mit Trimethylamin zu einer quaternären Stickstofffunktion umgesetzt. In Analogie zum Verfahren von Wendler et. al.²⁴ wurden die wasserunlöslichen Precursorpolymere in einer 20 %-igen wässrigen Triethylaminlösung dispergiert und unter Rückflussbedingungen bei 100 °C umgesetzt. Nach einer Reaktionszeit von 60 min wurde jeweils ein homogen gelöstes Produkt erhalten. Die Aufreinigung erfolgte durch Ultrafiltration (3 kD Membran) und die Isolation des Produkts mittels Gefriertrocknung. Mit Hilfe der ¹H-NMR Spektroskopie wurde gefunden, dass die Chlortrimethylgruppen quantitativ zu Trimethylammoniumgruppen umgesetzt worden waren. Bei den gewählten Reaktionsbedingungen erfolgte neben der Quaternisierung eine partielle Verseifung der Estergruppen des Methylmethacrylats, d.h. es entstanden jeweils Terpolymere aus quaternisiertem Vinylbenzylchlorid, Methylmethacrylatund Methylacrylsäure. Das Verhältnis von Methylmethacrylat und Methylacrylsäure wurde mittels infrarotspektroskopischer Untersuchungen bestimmt, womit sich die in Tabelle 2 aufgeführte chemischen Zusammensetzung der Polyelektrolyte ergibt.

### 1.3.3 Komplexierung der Polyelektrolyte

Zur Komplexierung wurden je 100 mg Polyelektrolyt in 100 ml Wasser gelöst und auf 90 °C erwärmt. Jeweils 1.05 Äquivalente Perfluordodekansäure (Aldrich), mit der Stöchiometrie bezogen auf die kationischen Ladungen des Polyelektrolyten, wurden in 100 ml Wasser dispergiert, zur Verbesserung des Lösungszustands mit NaOH auf pH 7 eingestellt und bei 90 °C für 30 min verrührt und zur Polyelektrolytlösung gegeben. Durch Vermischen von Polyelektrolyt- und Tensidlösung wurde das PEFA gebildet. Der Komplex des Polyelektrolyten, der keine Carbonsäuregruppen enthielt, fiel als Niederschlag spontan quantitativ aus der Lösung aus. Im Gegensatz dazu bilden die Komplexe derjenigen Polyelektrolyte, die Carbonsäuregruppen enthalten, Lösungen von Komplex-Nanopartikeln, die optisch klar sind und vergleichsweise niedrige Viskositäten im Vergleich zur optisch trüben und viskosen Lösung der eingesetzten reinen Perfluordodekansäure-Lösung besitzen.

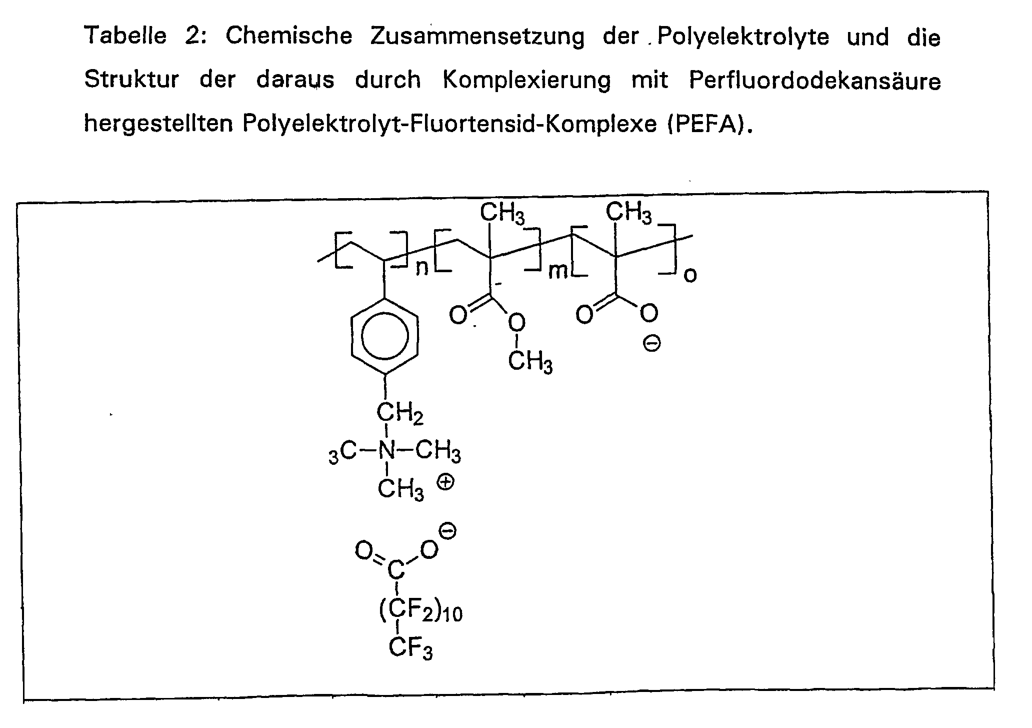

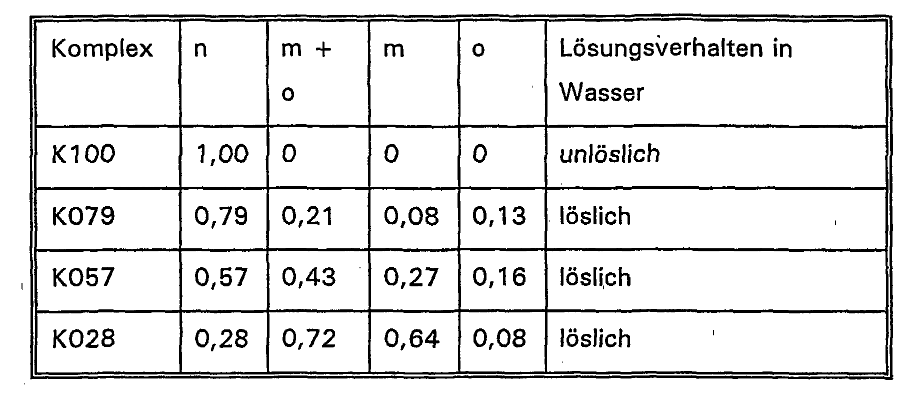

### 1.3.4 Strukturen der PEFA-Nanopartikel

Mit Hilfe der Röntgenkleinwinkelstreuung wurden 1,0 gewichtsprozentige wässrige Lösungen von Komplex-Nanopartikeln untersucht. Dabei wurden Streukurven gefunden wie sie für Nanopartikel mit Größen von kleiner 100 nm typisch sind. Die Auswertung der Streukurven erfolgte nach dem Standardverfahren von Glatter,^{25,26,27} exemplarisch zusammengefasst von Kaler et. al.²⁸ Es wurde gefunden, dass die Dispersion von Komplex K079 scheibenförmige Nanopartikel mit relativ einheitlicher Größe enthält, deren Durchmesser 30 nm ± 2 nm und deren Höhe 2,2 nm ± 0.2 nm beträgt. Die Dispersion von Komplex K057 enthält relativ einheitliche Nanopartikel mit Zylindergeometrie, deren Durchmesser 2,8 nm ± 0,2 nm und deren Höhe 20 nm ± 2 nm beträgt. Die Dispersion von Komplex K028 enthält Nanopartikel uneinheitlicher Größe von tendenziell zylinderförmiger Geometrie mit maximalen Ausdehnungen von 20 bis 40 nm.

### 2. Hämokompatibilität

Zur Abschätzung der Hämokompatibilität von PEFA müssen ihre Eigenschaften im Kontakt mit Blut in ihrer Gesamtheit betrachtet werden. Als entscheidende Parameter sind Gerinnungsaktivierung, Thrombogenität sowie Immunreaktionen wie insbesondere die Komplementaktivierung zu betrachten. Untersucht wurden a) vernetzte PEFAs (siehe Beispiel 1.1) und b.) komplexadditivierte Polyurethanlacke (siehe Beispiel 1.2).

Die Hämokompatibilität der auf ein Trägermaterial (Polytetrafluorethylen, Aluminium) aufgebrachten PEFA-Beschichtung wurde in vitro sowohl in einem statischen als auch in einem dynamischen Blutinkubationsversuch bestimmt. Bei der dynamischen Blutinkubation wird das Blut während der Inkubation durch Pumpbetrieb über der PEFA-Beschichtung zirkuliert, bei der statischen Inkubation wird die Sedimentation der Blutzellen während der Inkubation durch Rotation der Probenkammer verhindert.

### 2.1 Gerinnungsaktivität

Die Gerinnungsaktivität wurde durch Bildung von Thrombin (nachgewiesen als Thrombin-Antithrombin-Komplex (TAT)) nachgewiesen. Als Ausgangswert diente der Thrombingehalt der Probe vor der Inkubation. Polytetrafluorethylen und Glas dienten als Referenzmaterialien. Es wurde gefunden, dass die Gerinnungsaktivität der PEFA-Beschichtungen sehr gering ist und im Referenzbereich des in der Medizintechnik für Anwendungen im Blutkontakt verbreiteten Referenzmaterials Polytetrafluorethylen liegt.

### 2.2 Thrombozytenaktivität

Ein weiterer wichtiger Faktor bei der Einschätzung der Hämokompatibilität ist die Aktivierung der Thrombozyten. Die Bildung des Plättchenfaktors 4 (PF4) ist neben der quantitativen Abschätzung der Thrombozytenzahl ein wichtiges Indiz für diese Aktivierung. Das Ausmaß der Bildung von PF4 nach Inkubation in der statischen Kammer liegt für den Polysiloxankomplex WR1100-C12 (Komplexierungsgrad von 50 %) im Bereich des Referenzmaterials Polytetrafluorethylen. Gleiches gilt für den Komplex PEI-Zonyl FSA in Polyurethanlack.

### 2.3 Komplementaktivierung

Die Aktivierung der Komplementkaskade als ein Teil der Immunabwehr des Körpers wurde durch Bildung von Komplementfragment C5a bestimmt. Es wurde gefunden, dass die Komplementaktivierung des PEFA (WR1100-C12 mit einem Komplexierungsgrad von 50 %) leicht oberhalb der des Referenzmaterials Polytetrafluorethylens liegt. Die des Komplexes PEI-FSA in Polyurethanlack liegt im Bereich oder unterhalb der des Referenzmaterials Polytetrafluorethylen.

### 2.4 Hämolyse

Die Bestimmung der Hämolyse - bzw. der resultierenden Freisetzung von Hämoglobin aus Erythrozyten - ist ein weiterer wichtiger Hämokompatibilitätsparameter. Dabei wird die mechanische Zerstörung der Erythrozyten bzw. eine Erythrozytenvitalitätsstörung durch gelöste Komponenten überprüft. Durch keines der untersuchen PEFA-Materialien wurde im in vitro Versuch Hämolyse induziert. Untersucht wurden niederenergetische Polysiloxankomplexe, beschrieben in Beispiel 1.1 und PEFA-Kolloid additivierte Polyurethanfolien und -beschichtungen, beschrieben in Beispiel 1.2.

### 3. Zelladhäsion

Mit Hilfe von Zellkulturtests (Maus-Fibroblasten L929) wurde die Besiedlung von lebenden Zellen an verschiedenen PEFA Oberflächen untersucht. Experimentelle Details finden sich bei Morra und Cassinelli.²⁹

### 3.1 Polyethylenimin-Perfluorcarboxylat-Komplexe

Quarzscheiben (10 x 5 x 1 mm) wurden mit literaturbekannten Polyethylenimin-Perfluorcarboxylat-Komplexen (PEI-Cₙ) im Tauchverfahren beschichtet.³⁰ Die Kettenlänge des fluorierten Carboxylates betrug 4 (PEI-C4), 7 (PEI-C7), 10 (PEI-C10) und 12 (PEI-C12) Kohlenstoffatome. Die Quarzträger wurden in Petrischalen gegeben und mit 5 ml des Mediums RPM1-1640 (Sigma) inkubiert, das 200000 Zellen pro ml Maus-Fibroblasten der Zellinie L 939 enthielt. Weiterhin enthielt das Medium 1000 *µ*l des Antibiotikums GPS pro 100 ml. Die Besiedlung der Maus-Fibroblasten auf den Komplexoberflächen wurde mittels Lichtmikroskopie verfolgt.

Es wurde gefunden, dass bei allen Komplexoberflächen die Besiedlung gegenüber dem nichtbeschichteten Petrischalenboden stark reduziert ist. Anzeichen für eine toxische Wirkung der Oberflächen auf die Fibroblasten, wie eine verringerte Mobilität oder eine erhöhte Mortalität, waren nicht erkennbar. Es zeigte sich tendenziel sehr klar, dass das Ausmaß der Besiedlung von der Kettenlänge des fluorierten Carboxylates abhängt. Bei den Oberflächen der Komplexe PEI-C4 und PEI-C7 wurde nach 27 h ein erkennbares Ausbreiten der Fibroblasten vom Rand des Quarzträgers aus beobachtet. Bei PEI-C10 war nach 27 h eine randnahe Gruppenbildung von Fibroblasten festzustellen, während bei PEI-C12 nur sehr wenige ausgestreckte, d.h. siedelnde Zellen gefunden wurden.

### 3.2 PEFA-additivierte Polyurethane

Die Ergebnisse der Zellkulturtests (Maus-Fibroblasten L929) an Oberflächen des Komplexes PEI-Zonyl FSA in Polyurethanmatrix (siehe Beispiel 1.2) ergaben keine Adhäsion von Zellen an den Komplexoberflächen nach Kontaktzeiten, die auf dem Referenzmaterial (Tissue-Culture-Polystyren) zu konfluenter Besiedlung führten. Toxische Wirkungen der PEFA-Oberflächen auf die Zellen konnten nicht festgestellt werden.

### 4. Stabilisierung der Proteinstruktur

Mit Hilfe des Circular-Dichroismus (CD) wurde der Einfluss von PEFA-Kolloiden auf die Adsorption und die Sekundärstruktur von absorbiertem humanem Serum Albumin (HSA) untersucht.

**Tabelle 3:**

| Adsorption von HSA an Polysiloxan-Fluortensid-Komplexe | | | | |
|---|---|---|---|---|
| PEFA-Oberfläche | WR1100-C12 | WR1100-C12 | WR1100-C8 | WR1100-C8 |
| Grad der Komplexierung | 100 % | 50 % | 100 % | 50 % |
| | | | | |
| Adsorbierte Masse | 4,2 mg/m² | 1,3 mg/m² | 1,4 mg/m² | 1,5 mg/m² |
| | | | | |
| α-helix | 40 % | 35 % | 76 % | 64% |
| | | | | |
| β-sheet | 21 % | 23 % | 0 % | 4 % |
| | | | | |
| random coil | 39 % | 42 % | 24 % | 32 % |

### 4.1 Polyethylenimin-Perfluorcarboxylat-Komplexe

Quarzscheiben (10 x 5 x 1 mm) wurden mit literaturbekannten Polyethylenimin-Perfluorcarboxylat-Komplexen beschichtet.³¹ Die komplexbeschichteten Quarzscheiben wurden mit 1 x 10⁻⁶ molaren Lösungen von Human Serum Albumin (HSA) 2 h bei 22 °C inkubiert und anschließend die Menge an adsorbierten HSA mittels Circulardichroismus-Spektroskopie bestimmt. Es wurde gefunden, dass die Menge des adsorbierten HSA mit der Kettenlänge des fluorierten Carboxylats in der gleichen Reihenfolge abnahm wie die Oberflächenenergie der Beschichtung. Die adsorbierte Menge HSA betrug für Polyethylenimin-Perfluorbutanoat (PEI-C4) 5.9 mg m⁻², für Polyethylenimin-Perfluorheptanoat (PEI-C7) 4.1 g m⁻² und für Polyethylenimin-Perfluordecanoat 1.6 g m⁻². Für Polyethylenimin-Perfluordodecanoat (PEI-C 12) konnte keine Adsorption von HSA mehr nachgewiesen werden. Die Konformation des adsorbierten HSA wurde mittels Circulardichroismus-Spektroskopie verfolgt. Dabei wurde gefunden, dass das adsorbierte HSA an den PEI-C4 und PEIC7-Oberflächen vollständig und an den PEI-C10-Oberflächen weitgehend in seiner nativen Struktur mit α-helikaer Konformation erhalten blieb.

### 4.2 Polysiloxan-Fluortensid-Komplexe

Die Oberflächen von vernetzten Komplexen, wie sie aus aminofunktionalisierten Polysiloxanen und fluorierten Tensiden gebildet werden (siehe Beispiel 1.1), wurden mit HSA inkubiert. In allen Fällen wird nur eine geringe Menge von 1,3 bis 4,2 mg HSA adsorbiert. Weiterhin ist zu erkennen, dass der Anteil an α-helikaler Struktur hoch ist. Er beträgt bis zu 76 % (Komplex WR1100-C8 mit einem Komplexierungsgrad von 50 %). Weiterhin wurde festgestellt, dass zwischen Fluorkettenlänge und Komplexierungsgrad einerseits und der Proteinstruktur andererseits ein Zusammenhang besteht. Zudem steht zweifelsfrei fest, dass die PEFA-Komplexoberflächen einen konservierenden Effekt auf die native Struktur des HSA ausüben.

### 5. β-Amyloid-Rückfaltung und Auflösung von Amyloidablagerungen

### 5.1 Sekundärstruktur von Amyloid-Proteinen in Lösung

Amyloid (1-40) (BACHEM, Heidelberg; Reinheit > 96 %) wurde in entionisiertem Wasser zu einer 0,33 mMol Lösung gelöst und diese 3 Tage bei 37 °C inkubiert. Danach enthielt die Sekundärstruktur der Amyloid-Proteine 0 % α-Helix, 59 % β-Faltblatt und 41 % Random-Coil-Konformation. Die Konformationsbestimmung erfolgte circulardichroitisch bei 22 °C im Wellenlängenbereich von 190 bis 260 nm in üblicher Weise.³² Die quantitative Auswertung der circulardichroitischen Spektren erfolgte mit Hilfe des von Screerama und Woody beschriebenen Selcom-Programms.³³ Circulardichrotische Messungen wurden innerhalb einer Woche täglich wiederholt. Es wurde dabei gefunden, dass die Spektren und damit die Sekundärstruktur der Amyloid-Proteine ab dem 3. Tag konstant blieb.

### 5.2 Rückfaltung von Amyloid-Proteinen in Kontakt mit PEFA-Folie

In ein schmales Reagenzglas wurden 3 ml Amyloidiösung mit der in Beispiel 5.1 beschriebenen Sekundärstruktur gegeben und anschließend eine 5 mm x 30 mm große komplexhaltige Polymerfolie (hergstellt gemäß Beispiel 1.2) zugegeben. Die Konformation der Amyloid-Proteine wurde an 3 aufeinander folgenden Tagen circulardichroitisch bestimmt. Dabei wurde eine deutliche Veränderung der Konformation gefunden. Wie aus Tabelle 4 ersichtlich wird, verringert sich der Anteil an β-Faltblatt zugunsten des Anstiegs des Anteils an α-Helix.

**Tabelle 4:**

| Zeitliche Veränderung der Sekundärstruktur von Amyloid-Protein in Lösung bei Kontakt mit PEFA-additivierter Polyurethanfolie | | | |
|---|---|---|---|
| | 1. Tag | 2. Tag | 3. Tag |
| α-Helix | 0 % | 9 % | 20 % |
| β-Faltblatt | 60 % | 44 % | 38 % |
| β-Turn | 9 % | 16 % | 11 % |
| Random coil | 33 % | 31 % | 32 % |

Von einem an Amyloidose erkrankten Marder, sichtbar an Leber, Niere und Milz, wurden Gewebeproben entnommen. In vergleichbar große Gewebeproben der Niere von jeweils etwa 2 cm³, wurden jeweils 0,5 ml wässrige Nano-Dispersionen (hergestellt gemäß Beispiel 1.3) der PEFA 1, 2, bzw. 3 injiziert. Nach 3 h Einwirkzeit wurden die mit PEFA behandelten Gewebestücke zusammen mit gleichgroßen, aber unbehandelten Gewebestücken in 7-5 %-igem Formalin fixiert und in üblicher Weise³⁴ histologisch aufbereitet. 5 *µ*m starke Gewebeschnitte im Bereich der Nieren-Glumerula wurden mit Kongorot eingefärbt, nach der in der Histologie üblichen Prozedur für die Einfärbung von Amyloidablagerung in nativem Gewebe.³⁵

Bereits die Prüfung auf Sicht im Fluoreszenzmikroskop bei 40- und 100-facher Vergrößerung zeigte Unterschiede zwischen den nicht und den mit Fluorpolymer behandelten Gewebeproben: Deutlich zu erkennen war, dass durch die Behandlung mit PEFA-Nanodispersionen Anteile besonders aus den kompakteren Amyloidablagerungen abgebaut und herausgelöst worden waren.

Um diesen Abbau zu quantifizieren, wurde im Leica Spectral Confocal Microscope TCSSP bei 10-facher Vergrößerung unter konstant gehaltenen Messbedingungen für jede der aufgeführten Gewebeschnitte die Kongorotintensiät jeweils gleich großer Flächen (Flächenumfang jeweils 430 µm) mit vergleichbarer Strukturierung, gescannt. Es wurden die in Tabelle 5 dargestellten Zahlenwerte für die Kongorotintensität erhalten, die ein vergleichbares, relatives Maß für die Amyloidkonzentration sind.

**Tabelle 5:**

| Bestimmung der Auflösung von Amyloid-Ablagerungen in nativem Gewebe mittels Fluoreszenzmikroskopie nach der Behandlung mit PEFA-Nanodispersionen. Die Konzentration an Komplex betrug jeweils 1 Gew.-% in destilliertem Wasser. | | |
|---|---|---|
| Art der Gewebebehandlung | Fluoreszenzintensität der Amyloid-Ablagerung | |
| | von Gewebeflächen insgesamt Counts (normiert) | von kompakter Ablagerung Counts (normiert) |
| unbehandelt | 84098 (100 %) | 33741 (100 %) |
| | | |
| PEFA-Dispersion 1 | 49193 (58,5 %) | 1 8532 (54,9 %) |
| (K057 in Tabelle 2) | | |
| PEFA-Dispersion 2 | 16112 (19,2 %) | 11501 (34,1 %) |
| (K079 in Tabelle 2) | | |
| PEFA-Dispersion 3 | 32511 (38,7 %) | 24338 (72,1 %) |
| (K028 in Tabelle 2) | | |

### Literaturverzeichnis

¹ Übersichtsartikel: "Nano-structured materials with low surface energies formed by polyelectrolytes and fluorinated amphiphiles (PEFA)" A.F. Thünemann, *Polymer International* **2000***, 49,* 636-644
² H. Klinkmann, M. Davison, eds. Proceedings of the Consensus Conference on Biocompatibility. *Nephrol Dial Transplant* **9** (1994); Missirlis Y.F., Lemm W., eds. *Modern aspects ofprotein adsorption on biomaterials.* Dordrecht: Kluwer, 1991
³ T.S.J. Elliot, M.H. Faroqui, *Brit. J. Hosp. Med.* **48** 497 (1992); Gristina, A.G.; Hobgood, C.F., Webb, L.X., Myrvik, Q.N., *Biomater.* **8**, 423 (1987); Merritt, K., Gaind, A., Anderson, J.M., *Biomed. Mater. Res.* **39** (1998) 415
⁴ J.L. Brash, P. Wojchieowski, eds. *Interfacial phenomena and bioproducts.* New-York: Marcel Dekker, 1996; Ratner B.D., *J. Biomed. Mat. Res.* **27**, 837 (1993); Ragaller, M., Werner, C., Bleyl, J., Adam, S., Jacobasch, H.-J., Albrecht, D.M., *Kidney Intern.* **64** (1998) 84
⁵ P. Wiggins, J. Franke, H. Pudleiner, R. Dujardin, *Kunststoffe* **85** (1995) 9; Kennedy J., *J.M.S.- Pure Appl. Chem.* **A31** (1994) 177; Ikada, Y. in: *Interfacial Biocompatibility,* ACS Symposium Series 540 (1994) 35
⁶ P. Desai, J.A. Hubbell, *Biomaterials* 12 (1991) 144
⁷ R. LaPorte: *Hydrophilic Polymer coatings for medical devices.* Technomic, Lancaster, 1997
⁸ BMBF-Projekt 03N40017, BMBF-Projekt 03M1505
⁹ S.H. Sheth, D.Leckband, *Proc. Natl. Acad. Sci. USA* **94** (1997) 8399
¹⁰ BMBF-Projekt 03N4000; Vienken J., Diamantoglou M., Hahn C., Kamusewitz H., Paul D., *Artif. Organs* **19** (1995) 398
¹¹ BMBF-Projekt 03M1506; Vasilets, V.N., Hermel, G., König, U., Werner, C., Müller, M., Simon, F., Grundke, K., Ikada, Y., Jacobasch, H.-J., *Biomaterials* **18** (1997) 1139
¹² Iwasaki, Y., Mikami, A., Kurita, K., Yui, N., Ishihara, K., Nakabayashi, N., *J. Biomed. Mater. Res.* **36** (1997) 508
¹³ B. Jansen, *Int. J. Med. Microbiol.* **272** (1990) 401
¹⁴ A. Atala, D.J. Mooney, J.P. Vacanti, R. Langer, *Synthetic biodegradable polymer scaffolds.* Birkhäuser, Basel 1997
¹⁶ D.E. Ellis, I.V. Yannas, in: Wise, D.L., Trantolo, D.J., Altobelli, D.E. Yaszemski, M.J., Gresser, J.D. (eds.): *Human Biomaterials Applications.* Humana Press, New Jersey 1996. 415'
¹⁶ Vroman, L.; Leonhard, E.F. *Ann N. Y. Acad. Sci* **1977,** 283
¹⁷ A. Augsburg, K. Grundke, K. Pöschel, H.-J. Jacobasch, A.W. Neumann, *Acta Polym.,* 1998, **49**, 417
¹⁸ Li, D.; Neuman, A. J. *Cooloid Interface Sci.,* **1992**, *148*, 190
¹⁹ F. Schulman, W.A. Zisman, *J*. *Colloid Sci.,* 1952, **7**, 465
²⁰ F.M. Fowkes, *J*. *Phys. Chem.,* 1962, **66**, 382
²¹ L.A. Girifalco, R.J. Good, *J*. *Phys. Chem.,* 1957, **61**, 902
²² C.D. Bain, G.M. Whitesides, *J*. *Am. Chem. Soc.,* 1988, **110,** 5898
²³ R. Wagner, L. Richter, Y. Wu, J. Weißmüller, A. Kleewein, E. Hengge, *Applied Organometallic Chemistry* 1998, **12,** 265
²⁴ Wendler, U.; Bohrisch, J.; Jaeger, W.; rother, G.; Dautzenberg, H. *Macromolecular Rapid Commun.* **1998,** *19,* 185-190
²⁵ Glatter, O. *Acta Physica Austria* **1977,** *47*, 83
²⁶ Glatter, O. *J. Appl. Cryst.* **1977,** *10,* 415
²⁷ Glatter, O. *J. Appl. Cryst.* **1979**, *12,* 166
²⁸ lampietro, D.J.; Brasher, L.L.; Kaler, E.W.; Stradner, A.; Glatter, O. *J. Phys. Chem. B* **1998**, *102,* 3105-3113
²⁹ Morra, M,; Cassinelli, C. *Langmuir* **1999**, *15*, 4658-4663
³⁰ Thünemann, A.F. *Langmuir* **2000**, *16*, 824-828
³¹ Thünemann, A.F. *Langmuir* **2000**, *16,* 824-828
³² Johnson, W.C.; *Proteins: Structure, Function and Genetics* **1990,** *7*, 205-214
³³ Screerama, N.; Woody, R.W. *Analytical Biochem.* **1993,** *209,* 32
³⁴ Böck, P. in *Mikroskopische Technik,* Urban und Schwarzenberg: München, Wien, Baltimore, 1989
³⁵ SIGMA Diagnostics, Kongorot-Färbung (Amyloid-Nachweis) Katalog Nr. HT 60

## Patentansprüche

1. Biokompatible kolloidale Zusammensetzung umfassend
a) mindestens einen Polyelektrolyten, welcher kationische und anionische Gruppen oder/und Siloxaneinheiten enthält und
b) mindestens ein Fluor-enthaltendes Amphiphil.

2. Zusammensetzung nach Anspruch 1,
**dadurch gekennzeichnet,**
**dass** das Amphiphil mindestens eine -CF₃-Gruppe enthält.

3. Zusammensetzung nach Anspruch 1 oder 2,
**dadurch gekennzeichnet,**
**dass** das Amphiphil mindestens eine Gruppierung -(CF₂)ₙ- umfasst, worin n ≥ 3.

4. Zusammensetzung nach einem der vorhergehenden Ansprüche,a
**dadurch gekennzeichnet,**
**dass** das Amphiphil ausgewählt wird aus der Gruppe umfassend fluorierte Carboxylate, fluorierte Sulfonate, fluorierte Phosphate und Bis(fluoriertes Alkyl)-2-sulfosuccinate.

5. Zusammensetzung nach Anspruch 1,
**dadurch gekennzeichnet,**
**dass** es sich bei dem Polyelektrolyten um ein Polysiloxan mit 0,1 bis 100 Mol-% ionischen Gruppen handelt.

6. Zusammensetzung nach einem der vorhergehenden Ansprüche,
**dadurch gekennzeichnet,**
**dass** der Polyelektrolyt die Formel 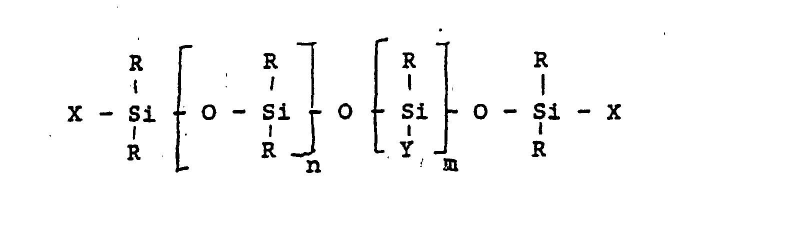 aufweist, worin R jeweils unabhängig Alkyl darstellt, X jeweils unabhängig Alkyl oder Hydroxy darstellt, Y einen Rest darstellt, der mindestens eine ionische oder ionisierbare Gruppe enthält,
n 0 bis 10.000 ist,
m 1 bis 10.000 ist und
n + m ≥ 50.

7. Zusammensetzung nach einem der vorhergehenden Ansprüche,
**dadurch gekennzeichnet,**
**dass** sie kationische und anionische Gruppen im Verhältnis von 100:1 bis 1:1 enthält.

8. Zusammensetzung nach einem der vorhergehenden Ansprüche,
**dadurch gekennzeichnet,**
**dass** der Polyelektrolyt 5 bis 100 Mol-% ionische Gruppen umfasst.

9. Zusammensetzung nach einem der vorhergehenden Ansprüche,
**dadurch gekennzeichnet,**
**dass** der Polyelektrolyt als kationische Gruppen quarternäre Amingruppen enthält.

10. Zusammensetzung nach einem der vorhergehenden Ansprüche,
**dadurch gekennzeichnet,**
**dass** der Polyelektrolyt als anionische Gruppen Carbonsäuregruppen enthält.

11. Zusammensetzung nach einem der vorhergehenden Ansprüche,
**dadurch gekennzeichnet,**
**dass** der mindestens eine Polyelektrolyt und das mindestens eine Amphiphil in Form von Komplexen vorliegen.

12. Biokompatibles Kolloid, gebildet aus einer Zusammensetzung nach einem der Ansprüche 1 bis 11.

13. Biokompatibles Kolloid nach Anspruch 12,
**dadurch gekennzeichnet,**
**dass** es in Form von in wässriger Lösung dispergierten Nanoteilchen vorliegt.

14. Biokompatibles Kolloid nach Anspruch 12,
**dadurch gekennzeichnet,**
**dass** es in Form eines Films oder einer Folie vorliegt.

15. Verfahren zur Herstellung eines biokompatiblen Kolloids nach Anspruch 14,
**dadurch gekennzeichnet,**
**dass** man
a) mindestens einen Polyelektrolyten, welcher kationische und anionische Gruppen oder/und Siloxaneinheiten enthält und
b) mindestens ein Fluor-enthaltendes Amphiphil zusammengibt und das Gemisch auf eine Temperatur von 120 °C bis 200 °C erwärmt.

16. Verfahren zur Herstellung eines biokompatiblen Kolloids nach Anspruch 13 umfassend die Schritte
a) Bereitstellen eines Polyelektrolyten, welcher kationische und anionische Gruppen enthält, und
b) Komplexieren des Polyelektrolyten mit einem Fluor-Amphiphilen in wässriger Lösung bei gleichzeitiger Dispergierung zu Nanopartikeln.

17. Verwendung einer biokompatiblen kolloidalen Zusammensetzung nach einem der Ansprüche 1 bis 11 oder/und eines biokompatiblen Kolloids nach einem der Ansprüche 12 bis 14 als Fäulnis-hemmendes Mittel.

18. Verwendung eines biokompatiblen Kolloids, gebildet aus mindestens einem Polyelektrolyten, welcher kationische und anionische Gruppen oder/und Siloxaneinheiten enthält, und mindestens einem Fluorenthaltenden Amphiphil zur Herstellung eines Gegenstandes mit einer hohen Hämokompatibilität oder/und einer geringen Zelladhäsion.

19. Verwendung eines biokompatiblen Kolloids umfassend mindestens einen Polyelektrolyten, welcher kationische und anionische Gruppen oder/und Siloxaneinheiten enthält, und mindestens ein Fluor-enthaltendes Amphiphil zur Stabilisierung von Proteinstrukturen, zur Rückfaltung von Proteinen oder/und zur Auflösung von Amyloidablagerungen.

## Revendications

1. Composition colloïdale biocompatible comprenant
a) au moins un polyélectrolyte qui contient des groupes cationiques et anioniques et/ou des unités de siloxane et
b) au moins un amphiphile contenant du fluor.

2. Composition selon la revendication 1 **caractérisée en ce que** l'amphiphile contient au moins un groupe -CF₃.

3. Composition selon la revendication 1 ou 2 **caractérisée en ce que** l'amphiphile comprend au moins un groupement -(CF₂)ₙ- où n ≥ 3.

4. Composition selon l'une des revendications précédentes **caractérisée en ce que** l'amphiphile est choisi dans le groupe comprenant les carboxylates fluorés, les sulfonates fluorés, les phosphates fluorés et les bis(alkyle fluoré)-2-sulfosuccinates.

5. Composition selon la revendication 1 **caractérisée en ce que** le polyélectrolyte est un polysiloxane ayant 0,1 à 100 mol% de groupes ioniques.

6. Composition selon l'une des revendications précédentes **caractérisée en ce que** le polyélectrolyte présente la formule 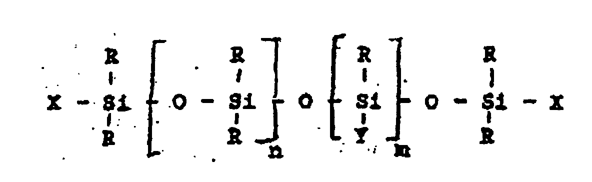 où R représente chaque fois indépendamment alkyle, X représente chaque fois indépendamment alkyle ou hydroxyle, Y représente un groupement qui contient au moins un groupe ionique ou ionisable,
n est 0 à 10000,
m est 1 à 10000 et
n + m *≥* 50*.*

7. Composition selon l'une des revendications précédentes **caractérisée en ce qu'**elle contient des groupes cationiques et anioniques dans le rapport de 100:1 à 1:1.

8. Composition selon l'une des revendications précédentes **caractérisée en ce que** le polyélectrolyte comprend 5 à 100 mol% de groupes ioniques.

9. Composition selon l'une des revendications précédentes **caractérisée en ce que** le polyélectrolyte contient des groupes amine quaternaires comme groupes cationiques.

10. Composition selon l'une des revendications précédentes **caractérisée en ce que** le polyélectrolyte contient des groupes acide carboxylique comme groupes anioniques.

11. Composition selon l'une des revendications précédentes **caractérisée en ce que** le ou les polyélectrolytes et le ou les amphiphiles sont sous forme de complexes.

12. Colloïde biocompatible formé à partir d'une composition selon l'une des revendications 1 à 11.

13. Colloïde biocompatible selon la revendication 12 **caractérisé en ce qu'**il est sous forme de nanoparticules dispersées en solution aqueuse.

14. Colloïde biocompatible selon la revendication 12 **caractérisé en ce qu'**il est sous forme d'un film ou d'une feuille.

15. Procédé de préparation d'un colloïde biocompatible selon la revendication 14 **caractérisé en ce que** l'on mélange
a) au moins un polyélectrolyte qui contient des groupes cationiques et anioniques et/ou des unités de siloxane et
b) au moins un amphiphile contenant du fluor et on chauffe le mélange à une température de 120°C à 200°C.

16. Procédé de préparation d'un colloïde biocompatible selon la revendication 13 comprenant les étapes de
a) préparation d'un polyélectrolyte qui contient des groupes cationiques et anioniques, et
b) complexation du polyélectrolyte avec un amphiphile fluoré en solution aqueuse et dispersion simultanée en nanoparticules.

17. Utilisation d'une composition colloïdale biocompatible selon l'une des revendications 1 à 11 et/ou d'un colloïde biocompatible selon l'une des revendications 12 à 14 comme agent inhibant la décomposition.

18. Utilisation d'un colloïde biocompatible formé à partir d'au moins un polyélectrolyte, qui contient des groupes cationiques et anioniques et/ou des unités de siloxane, et d'au moins un amphiphile contenant du fluor pour la production d'un objet ayant une haute hémocompatibilité et/ou une faible adhésion cellulaire.

19. Utilisation d'un colloïde biocompatible comprenant au moins un polyélectrolyte, qui contient des groupes cationiques et anioniques et/ou des unités de siloxane, et au moins un amphiphile contenant du fluor pour la stabilisation de structures protéiques, pour le repliement de protéines et/ou pour la désagrégation de dépôts d'amyloïde.

## Claims

1. Biocompatible colloidal composition comprising
a) at least one polyelectrolyte which contains cationic and anionic groups or/and siloxane units and
b) at least one fluorine-containing amphiphile.

2. Composition as claimed in claim 1,
**characterized in that**
the amphiphile contains at least one -CF₃- group.

3. Composition as claimed in claim 1 or 2,
**characterized in that**
the amphiphile contains at least one -(CF₂)ₙ- group in which n ≥ 3.

4. Composition as claimed in one of the previous claims,
**characterized in that**
the amphiphile is selected from the group comprising fluorinated carboxylates, fluorinated sulphonates, fluorinated phosphates and bis(fluorinated alkyl)-2-sulphosuccinates.

5. Composition as claimed in claim 1,
**characterized in that**
the polyelectrolyte is a polysiloxane containing 0.1 to 100 mole % ionic groups.

6. Composition as claimed in one of the previous claims,
**characterized in that**
the polyelectrolyte has the formula 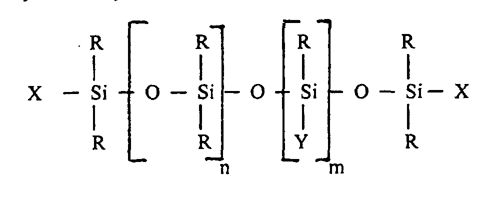 in which R in each case independently represents alkyl, X in each case independently represents alkyl or hydroxy, Y represents a residue which contains at least one ionic or ionizable group,
n is 0 to 10,000
m is 1 to 10,000 and
n+m ≥ 50.

7. Composition as claimed in one of the previous claims,
**characterized in that**
it contains cationic and anionic groups in a ratio of 100:1 1 to 1:1.

8. Composition as claimed in one of the previous claims,
**characterized in that**
the polyelectrolyte contains 5 to 100 mole % ionic groups.

9. Composition as claimed in one of the previous claims,
**characterized in that**
the polyelectrolyte contains quarternary amine groups as cationic groups.

10. Composition as claimed in one of the previous claims,
**characterized in that**
the polyelectrolyte contains carboxylic acid groups as anionic groups.

11. Composition as claimed in one of the previous claims,
**characterized in that**
the at least one polyelectrolyte and the at least one amphiphile are present in the form of complexes.

12. Biocompatible colloid formed from a composition as claimed in one of the claims 1 to 11.

13. Biocompatible colloid as claimed in claim 12,
**characterized in that**
it is present in the form of nanoparticles dispersed in aqueous solution.

14. Biocompatible colloid as claimed in claim 12,
**characterized in that**
it is present in the form of a film or a foil.

15. Process for producing a biocompatible colloid as claimed in claim 14,
**characterized in that**
a) at least one polyelectrolyte which contains cationic and anionic groups or/and siloxane units and
b) at least one fluorine-containing amphiphile are added together and the mixture is heated to a temperature of 120°C to 200°C.

16. Process for producing a biocompatible colloid as claimed in claim 13 comprising the steps
a) providing a polyelectrolyte which contains cationic and anionic groups and
b) complexing the polyelectrolyte with a fluorine-amphiphile in aqueous solution while simultaneously dispersing to form nanoparticles.

17. Use of a biocompatible colloidal composition as claimed in one of the claims 1 to 11 or/and a biocompatible colloid as claimed in one of the claims 12 to 14 as an anti-putrefactive agent.

18. Use of a biocompatible colloid formed from at least one polyelectrolyte which contains cationic and anionic groups or/and siloxane units and at least one fluorine-containing amphiphile to produce an object having a high haemocompatibility or/and a low cell adhesion.

19. Use of a biocompatible colloid comprising at least one polyelectrolyte which contains cationic and anionic groups or/and siloxane units and at least one fluorine-containing amphiphile to stabilize protein structures, to refold proteins and/or to dissolve amyloid deposits.
